# EUROPEAN PATENT SPECIFICATION

(11) **EP 3 944 975 B1**
(45) Date of publication and mention of the grant of the patent: **22.02.2023**
(21) Application number: 21185751.1
(22) Date of filing: 15.07.2021
(51) Int. Cl.: B60H 3/00, A61L 2/10, A61L 2/24, A61L 9/20

(54) **ULTRAVIOLET STERILIZATION APPARATUS FOR A VEHICLE**
VORRICHTUNG ZUR ULTRAVIOLETTEN STERILISATION EINES FAHRZEUGS
APPAREIL DE STÉRILISATION AUX ULTRAVIOLETS POUR UN VÉHICULE

(30) Priority: 28.07.2020 KR 20200093900
(43) Date of publication of application: 02.02.2022
(73) Proprietor: Kyungshin Co., Ltd., Incheon 21999 (KR)
(72) Inventor: HWANG, Chung Yul, Incheon 21999 (KR); KIM, Chang Suk, Incheon 21999 (KR); SUNG, Nak Sun, Incheon 21999 (KR); CHOI, Yun Suk, Incheon 21999 (KR)
(74) Representative: Algemeen Octrooi- en Merkenbureau B.V.

(56) References cited:
- DE-A1-102016 110 547
- KR-A- 20150 017 544
- KR-A- 20150 124 672

## Description

### CROSS-REFERENCE TO RELATED APPLICATION

This application claims priority to and the benefit of Korean Patent Application No. 10-2020-0093900, filed on July 28, 2020.

### BACKGROUND

### 1. Field of the Invention

The present invention relates to an ultraviolet sterilization apparatus for a vehicle, and more particularly, to an ultraviolet sterilization apparatus for a vehicle as outlined in the preamble of claim 1.

### 2. Discussion of Related Art

In general, air conditioners for vehicles have the functions of constant temperature and constant humidity to keep constant temperature and constant humidity as well as functions such as dehumidification, humidification, purification, ventilation, aroma, in addition to cooling and heating, which are the main functions so as to maintain the indoor air environment of the vehicle in a comfortable or suitable condition for the driver.

In such air conditioners for vehicles, since air is introduced into the interior of the vehicle through a cabin filter that filters and purifies pollutants such as exhaust gas, dust, and yellow dust in the air, the cabin filter plays an important role in whether indoor air is comfortable or not.

However, since the cabin filter is usually located inside a glove box in front of a passenger seat, when the cabin filter is inspected and replaced, the glove box needs to be opened, separated, and then reinserted. Also, since the cabin filter needs to be mounted in a predetermined direction in which the air flows, i.e., according to directivity, it is very cumbersome and laborious for the driver to replace the cabin filter from time to time, and it is difficult to immediately check a contamination state, so it takes quite a long time, and it is not easy to carry out.

The background technology of the present invention is disclosed in Korean Patent Laid-open Publication No. 10-2020-0075412 (published on June 26, 2020, Air Cleaner for Vehicle).

Korean Patent Publication No. KR2015 0017544 A discloses a sterilizer for a vehicle including a light-emission module unit disposed inside a vehicle to release ultraviolet light having sterilizing power; and a light-emission control unit for controlling at least one of a start point, wherein ultraviolet light is released from the light-emission module unit to sterilize indoor of a vehicle, and an end point wherein the sterilization is stopped, as well as the intensity of the ultraviolet light.

Korean Patent Publication No. KR2015 0124672 discloses an air purifying apparatus comprising an air purifier mounted inside a vehicle; a reservation setup part structured to receive reservation information including at least one among operation starting information and operation time information of the air purifier, and structured to reserve operation of the air purifier based on the reservation information; and a control part for controlling the operation of the air purifier based on the reservation information.

German Patent Publication No. DE102016110547 A1 discloses a device for disinfection of a car comprising a first electrode mostly covering one part of a transmission panel as well as multiple printed LEDs suspended in a semi-conductor oil ink of the first electrode and used for emitting and disinfection as well as a second electrode electrically connected with the multiple LEDs and wherein a light transmitting emitting layer is connected with the second electrode and/or one or more intermeddle layers configured to be an inner surface of a glass panel.

On the other hand, since the air contains various pollutants such as bacteria, viruses, mold, fine dust, harmful gases and odor components that are harmful to health, the demand for air purifiers for vehicles having an air purification function using a filter and a drying function using a blower so as to improve and maintain the indoor air quality of a vehicle has been increased.

Recently, air purifiers have been developed to control bacteria and purify indoor air by applying the sterilization effect of an ultraviolet (UV) light emitting diode (LED) that emits UV rays for air purification.

In particular, as large-scale infectious diseases (SARS / H1N1 flu / MERS / Corona 19, etc.) continue to occur, interest in sterilization and disinfection is also increasing, and the need for sterilization is increasing even in automobiles that are necessary for daily life.

### SUMMARY OF THE INVENTION

In accordance with an aspect of the present invention there is provided an ultraviolet sterilization apparatus for a vehicle, as set out in independent claim 1.

The UV sterilization apparatus for the vehicle may further include an operation display unit configured to display an operating state and result of the control unit.

The UV driving unit may include a plurality of ports which allow driving power to be output differently according to the UV modules.

The control unit may determine a case where an absence state of a passenger is maintained for more than a set time, as the operating condition based on any one or more of a starting state, a shifting state, a door opening/closing state, a door locking state, a seating state, an audio/video/navigation (AVN) state, an emergency light lighting state, a seat movement state, an fob signal state, and a rear seat detection state from the vehicle information.

When the control unit determines the operating condition and then a charging state of the main battery is less than expected driving power, the control unit may switch the power conversion unit to the auxiliary power so as to drive the UV driving unit.

When an abnormality occurs in the state of the main power input while the UV driving unit is operating, the control unit may switch the power conversion unit to the auxiliary power so as to operate the UV driving unit.

When the control unit determines the operating condition and then a charging state of the main battery is less than a set value, the control unit may stop an operation of the UV driving unit.

When a door lock is released while the UV driving unit is operating, the control unit may stop the operation of the UV driving unit and may temporarily stop counting of the sterilization driving time, and when the operating condition is satisfied again, the control unit may count the sterilization driving time so as to operate the UV driving unit again.

When the control unit determines again whether the operating condition is satisfied and then the operating condition is not satisfied, the control unit may stop the operation of the UV driving unit and may initialize counting.

The sterilization driving time may be set based on any one or more of a starting time, a door opening time, and temperature and humidity inside the vehicle.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a block diagram illustrating an ultraviolet sterilization apparatus for a vehicle according to an embodiment of the present invention;
FIG. 2 is an exemplary diagram illustrating installation positions of ultraviolet (UV) modules in the UV sterilization apparatus for a vehicle according to an embodiment of the present invention; and
FIG. 3 is a flowchart illustrating a method of controlling a UV sterilization apparatus for a vehicle according to an embodiment of the present invention.

### DETAILED DESCRIPTION OF EXEMPLARY EMBODIMENTS

As is traditional in the corresponding field, some exemplary embodiments may be illustrated in the drawings in terms of functional blocks, units, and/or modules. Those of ordinary skill in the art will appreciate that these block, units, and/or modules are physically implemented by electronic (or optical) circuits such as logic circuits, discrete components, processors, hard-wired circuits, memory elements, wiring connections, and the like. When the blocks, units, and/or modules are implemented by processors or similar hardware, they may be programmed and controlled using software (e.g., code) to perform various functions discussed herein. Alternatively, each block, unit, and/or module may be implemented by dedicated hardware or as a combination of dedicated hardware to perform some functions and a processor (e.g., one or more programmed processors and associated circuitry) to perform other functions. Each block, unit, and/or module of some exemplary embodiments may be physically separated into two or more interacting and discrete blocks, units, and/or modules without departing from the scope of the inventive concept. Further, blocks, units, and/or module of some exemplary embodiments may be physically combined into more complex blocks, units, and/or modules without departing from the scope of the inventive concept.

Hereinafter, an ultraviolet sterilization apparatus for a vehicle according to the present invention will be described with reference to the accompanying drawings. In this process, the thicknesses of lines or the sizes of components shown in the drawings may be exaggerated for clarity and convenience of description. In addition, terms to be described below are terms defined in consideration of functions in the present invention, which may vary according to the intention or custom of users or operators. Therefore, definitions of these terms should be made based on the contents throughout the present specification.

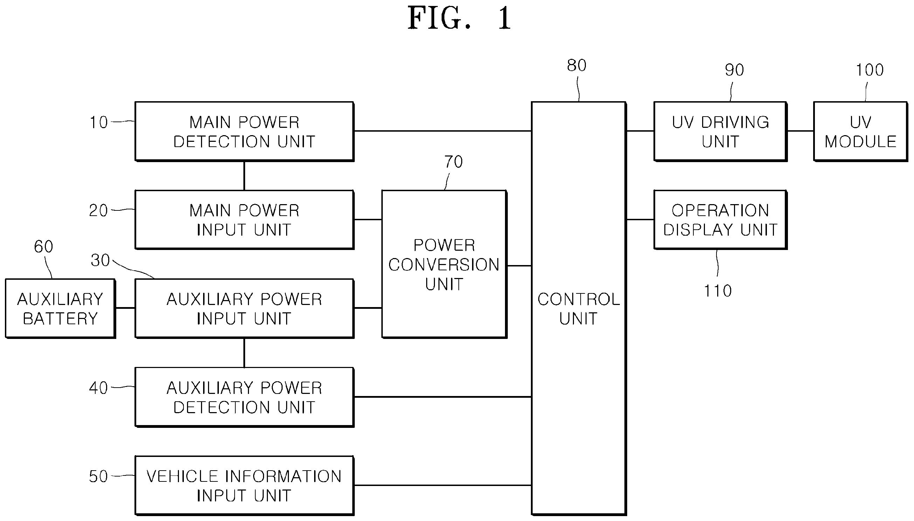

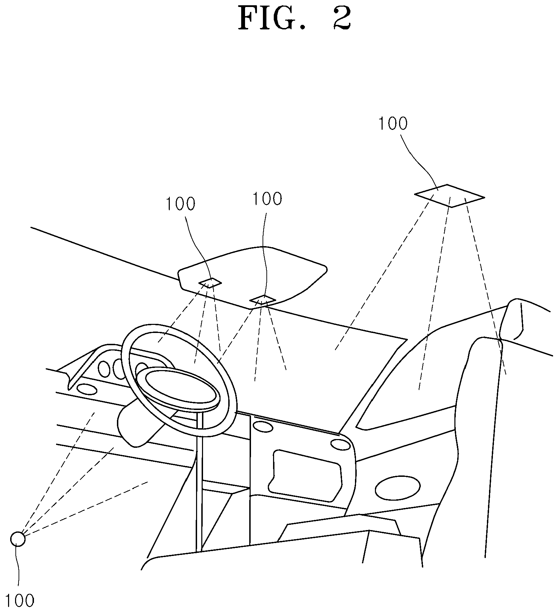

FIG. 1 is a block diagram showing an ultraviolet sterilization apparatus for a vehicle according to an embodiment of the present invention, and FIG. 2 is an exemplary view showing installation positions of ultraviolet (UV) modules in the UV sterilization apparatus for the vehicle according to an embodiment of the present invention.

As shown in FIG. 1, the UV sterilization apparatus according to the embodiment of the present invention may include a main power input unit 20, an auxiliary power input unit 30, a main power detection unit 10, an auxiliary power detection unit 40, a vehicle information input unit 50, a power conversion unit 70, a plurality of UV modules 100, an UV driving unit 90, and a control unit 80, as well as an operation display unit 110.

The main power input unit 20 provides a port for receiving main power supplied from a main battery of a vehicle.

The auxiliary power input unit 30 provides a port for receiving auxiliary power from an auxiliary battery 60.

The main power detection unit 10 may detect a state of main power input from the main power input unit 20.

The auxiliary power detection unit 40 may detect the charging state of the auxiliary battery 60 connected to the auxiliary power input unit 30.

The vehicle information input unit 50 may receive vehicle information for determining the state of the vehicle through vehicle internal communication.

Here, the vehicle information includes at least one of a starting state, a shifting state, a door opening/closing state, a door locking state, a seating state, an audio/video/navigation (AVN) state, an emergency light lighting state, a seat movement state, an fob signal state, a rear seat detection state, and the charging state of the main battery.

The power conversion unit 70 switches between main power and auxiliary power input from the main power input unit 20 and the auxiliary power input unit 30, respectively, so that power can be stably supplied.

The plurality of UV modules 100 include UV light emitting didoes (LEDs), and are installed at a plurality of locations in the vehicle to irradiate UV rays and sterilize the interior of the vehicle.

Here, the plurality of UV modules 100 may be installed to irradiate UV rays to the interior at a location of a central interior light or a front interior light of the ceiling of the vehicle so that UV rays can be irradiated without a shaded area inside the vehicle, as shown in FIG. 2, and the plurality of UV modules 100 may be installed to irradiate UV rays from the lower space of a driver's seat or passenger seat to the upper side and may be installed to irradiate UV rays from the left and right sides of the vehicle to the inside.

The UV driving unit 90 is provided with a plurality of ports to independently drive the UV modules 100, so that the driving power can be output differently or the driving can be performed for different times according to the installation location of the plurality of UV modules 100.

The control unit 80 receives vehicle information input from the vehicle information input unit 50, the state of main power, and the charging state, determines the operating condition of the UV modules 100, and controls a power switching unit to drive the UV driving unit 90 during a sterilization driving time.

Here, the sterilization driving time may be set based on any one or more of a starting time, a door opening time, and temperature and humidity inside the vehicle.

The control unit 80 may determine the case where the absence state of the passenger is maintained for more than a set time, as an operating condition based on a starting state, the shifting state, the door opening/closing state, the door locking state, the seating state, the AVN state, the emergency light lighting state, the seat movement state, the fob signal state and the rear seat detection state from the vehicle information.

In this way, the control unit 80 determines the absence state of the driver by determining whether an engine is turned off, is switched to a parking mode, a door is closed, the door is locked and no weight is detected on the seat from the vehicle information. In addition, the control unit 80 determines the absence state of the passenger by determining whether, additionally, the AVN state is an activated state, emergency lights are turned on, seats are moving, fob signals are detected inside the vehicle, and a rear seat detection state is input, and the control unit 80 determines the case where the absence state of the passenger is maintained for more than a set time, as an operating condition, so that the safety of the passenger due to UV sterilization can be considered as much as possible.

Thereafter, when the state of the main power input is normal after the control unit 80 determines the operating condition, the control unit 80 determines the charging state of the auxiliary battery 60, and when the charging state of the auxiliary battery 60 is greater than or equal to predicted driving power, the control unit 80 switches the power conversion unit 70 to the main power so as to operate the UV driving unit 90, thereby determining the secured state of the driving power and then stably operating the sterilization apparatus even when an abnormality of the main power occurs during the operation of the sterilization apparatus.

In addition, the control unit 80 determines the operating condition, and when the charging state of the main battery is less than the expected driving power, or when an abnormality occurs in the state of the main power while the UV driving unit 90 is operating, the control unit 80 switches the power conversion unit 70 to auxiliary power so as to operate the UV driving unit 90, thereby stably operating the operation of the sterilization apparatus.

Meanwhile, when the control unit 80 determines the operating condition and then the charging state of the main battery is less than a set value, the control unit 80 may stop the operation so as not to affect the operation of the vehicle.

In addition, when the door lock is released while the UV driving unit 90 is operating, the control unit 80 stops the operation of the UV driving unit 90 for safety and temporarily stops counting of the sterilization driving time, and when the operating condition is satisfied again, the control unit 80 counts the sterilization driving time so as to operate the UV driving unit 90 again, so that power consumption due to repeated sterilization can be reduced.

On the other hand, the control unit 80 temporarily stops the UV driving unit 90 and then determines whether the operation condition is satisfied again, and initializes the counting when the operating condition is not satisfied.

The operation display unit 110 displays the operating state and result of the control unit 80 so that the driver can visually check the operating state or operation result of the sterilization apparatus.

For example, the operation or completion state can be indicated through the color of an LED lamp, the progress state or progress result can be displayed sequentially by a plurality of LED lamps, and the operating state of the sterilization apparatus can be visually displayed by scanning a laser beam.

As described above, according to the UV sterilization apparatus for a vehicle according to the embodiment of the present invention, a plurality of UV LEDs installed inside the vehicle are independently driven to uniformly sterilized the interior space without a shaded area and the driving state of the vehicle and the state of the passenger are determined by receiving vehicle information so that the operation of the plurality of LEDs is controlled and discharge of the vehicle is prevented by utilizing an auxiliary battery.

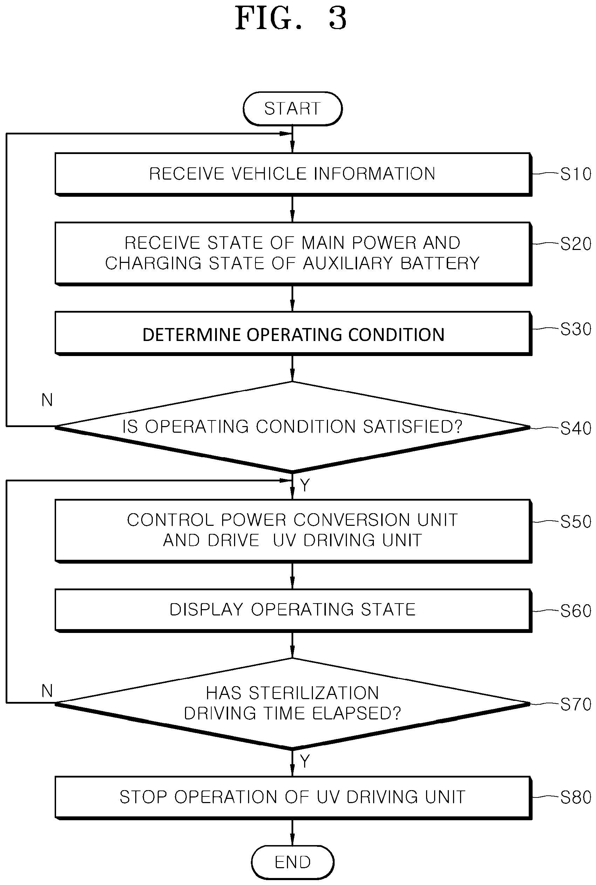

FIG. 3 is a flowchart illustrating a method of controlling a UV sterilizing apparatus for a vehicle according to an embodiment of the present invention.

As shown in FIG. 3, in the method of controlling a UV sterilization apparatus for a vehicle according to the embodiment of the present invention, first, the control unit 80 receives vehicle information for determining the state of the vehicle from the vehicle information input unit 50 (S10).

In addition, the control unit 80 receives the state of the main power and the charging state of the auxiliary battery 60 from the main power detection unit 10 and the auxiliary power detection unit 40, respectively (S20).

After receiving the vehicle information, the state of the main power, and the charging state of the auxiliary battery 60 through operations S10 and S20, the control unit 80 determines the operating condition of the UV modules 100 based on the input vehicle information, the state of the main power, and the charging state of the auxiliary battery 60.

That is, the control unit 80 may determine the case where the absence state of the passenger is maintained for more than a set time, as an operating condition based on a starting state, a shifting state, a door opening/closing state, a door locking state, a seating state, an AVN state, an emergency light lighting state, a seat movement state, an fob signal state and a rear seat detection state from the vehicle information.

In this way, the control unit 80 determines the absence state of the driver by determining whether an engine is turned off, is switched to a parking mode, a door is closed, the door is locked and no weight is detected on the seat from the vehicle information. In addition, the control unit 80 determines the absence state of the passenger by determining whether, additionally, the AVN state is an activated state, emergency lights are turned on, seats are moving, fob signals are detected inside the vehicle, and a rear seat detection state is input, and the control unit 80 determines the case where the absence state of the passenger is maintained for more than a set time, as an operating condition, so that the safety of the passenger due to UV sterilization can be considered as much as possible.

After the operating condition of the UV modules 100 is determined in operation S30, the control unit 80 determines whether the operating condition is satisfied (S40).

When it is determined that the operating condition is not satisfied in operation S40 of determining whether the operating condition is satisfied, the control unit 80 returns to operation S10 and may repeatedly determine the operating condition of the UV modules 100 by receiving the state of main power and the charging state of the auxiliary battery 60. In this case, the above process may be repeated only for a predetermined time after the engine is turned off so as to prevent power consumption.

When it is determined that the operating condition is satisfied in operation S40 of determining whether the operating condition is satisfied, the control unit 80 controls the power conversion unit 70 so as to operate the UV driving unit 90 during a sterilization driving time (S50).

Here, when operating the UV driving unit 90, the control unit 80 may differently adjust the operating time according to installation locations of the plurality of UV modules 100.

Also, when the state of the main power is normal, the control unit 80 determines the charging state of the auxiliary battery 60, and when the charging state of the auxiliary battery 60 is greater than or equal to the expected driving power, the control unit 80 switches the power conversion unit 70 to the main power so as to operate the UV driving unit 90, thereby determining the secured state of the driving power and then stably operating the sterilization apparatus even when an abnormality of the main power occurs during the operation of the sterilization apparatus.

In addition, when the charging state of the main battery is less than the expected driving power, or when an abnormality occurs in the state of main power while the UV driving unit 90 is operating, the control unit 80 switches the power conversion unit 70 to auxiliary power so as to operate the UV driving unit 90, thereby stably operating the operation of the sterilization apparatus.

Meanwhile, when the charging state of the main battery is less than a set value, the control unit 80 may stop the operation so as not to affect the operation of the vehicle.

In this case, the control unit 80 displays the operating state and result through the operation display unit 110 so that the driver can visually check the operating state or operation result of the UV driving unit 90 (S60).

For example, the operation or completion state can be indicated through the color of an LED lamp, the progress state or progress result can be displayed sequentially by a plurality of LED lamps, and the operating state of the UV driving unit 90 can be visually displayed by scanning a laser beam.

In this way, after operating the UV driving unit 90, the control unit 80 counts a sterilization driving time to determine whether the sterilization driving time has elapsed (S70).

When it is determined that the sterilization driving time has elapsed in operation S70 of determining whether the sterilization driving time has elapsed, the method returns to operation S50, and the UV driving unit 90 is operated during the sterilization driving time so that sterilization of the inside of the vehicle can be performed.

Here, the sterilization driving time may be set based on any one or more of a starting time, a door opening time, temperature and humidity inside the vehicle.

On the other hand, when it is determined that the sterilization driving time has elapsed in operation S70 of determining whether the sterilization driving time has elapsed, the control unit 80 stops the operation of the UV driving unit 90, initializes the counting and terminates the operation of the UV driving unit 90 (S80).

Meanwhile, when a door lock is released while the UV driving unit 90 is operating, the control unit 80 stops the operation of the UV driving unit 90 for safety and temporarily stops counting of the sterilization driving time, and when the operating condition is satisfied again, the sterilization driving time is counted and the UV driving unit 90 is operated again, so that power consumption due to repeated sterilization can be reduced. On the other hand, after temporarily stopping counting of the sterilization driving time, the control unit 80 determines whether the operation condition is satisfied again, and when the operation condition is not satisfied, the control unit 80 initializes counting of the sterilization driving time and then terminates the operation of the UV driving unit 90.

As described above, according to the method of controlling a UV sterilization device for a vehicle according to the embodiment of the present invention, a plurality of UV light emitting diodes (LEDs) installed inside the vehicle are independently driven to uniformly sterilized the interior space without a shaded area and the driving state of the vehicle, and the state of a passenger are determined by receiving vehicle information so that the operation of the plurality of LEDs is controlled and discharge of the vehicle is prevented by utilizing an auxiliary battery.

The implementations described herein may be implemented in, for example, a method or process, an apparatus, a software program, a data stream or a signal. Although discussed only in the context of a single form of implementation (e.g., only as a method), the implementation of the discussed features may also be implemented in other forms (e.g., an apparatus or program). The device may be implemented with appropriate hardware, software and firmware. The method may be implemented in an apparatus such as a processor, which generally refers to a processing device including, for example, a computer, a microprocessor, an integrated circuit or a programmable logic device, or the like. Processors also include communication devices such as computers, cell phones, personal digital assistants ("PDAs") and other devices that facilitate communication of information between end-users.

In the UV sterilization apparatus for a vehicle according to the aspect of the present invention, a plurality of LEDs installed inside the vehicle are independently driven so that the interior space can be uniformly sterilized without a shaded area and the driving state of the vehicle, and the state of a passenger are determined by receiving vehicle information so that the operation of the plurality of LEDs is controlled and discharge of the vehicle is prevented by utilizing an auxiliary battery.

While one or more embodiments have been described with reference to the figures, it will be understood by those of ordinary skill in the art that various changes in form and details may be made therein without departing from the scope of the present invention as defined by the following claims.

## Claims

1. An ultraviolet, UV, sterilization apparatus for a vehicle, comprising:
a main power input unit (20) configured to receive main power from a main battery of a vehicle;
a main power detection unit (10) configured to detect a state of a main power input from the main power input unit (20);
a vehicle information input unit (50) configured to receive vehicle information for determining a state of the vehicle;
a plurality of UV modules (100) configured to be installed at a plurality of locations in the vehicle and configured to irradiate UV rays and sterilize the interior of the vehicle during a sterilization driving time;
an UV driving unit (90) configured to drive the plurality of UV modules (100); and
a control unit (80) configured to receive a vehicle information input from the vehicle information input unit (50) and the state of the main power input, and
**characterized in that** the UV sterilization apparatus further comprises an auxiliary power input unit (30) configured to receive auxiliary power from an auxiliary battery (60), an auxiliary power detection unit (40) configured to detect a charging state of the auxiliary battery (60) connected to the auxiliary power input unit (30), and a power conversion unit (70) configured to switch between the main power input and an auxiliary power input from the main power input unit (20) and the auxiliary power input unit (30), respectively, the control unit (80) is further configured to receive the charging state of the auxiliary battery (60) and control the power conversion unit (70) so as to drive the plurality of UV modules (100) independently through the UV driving unit (90) during the sterilization driving time, and
when the state of the main power input is normal after the control unit (80) is configured to determine the operating condition of the UV modules (100), the control unit (80) is further configured to determine the charging state of the auxiliary battery (60), and when the charging state of the auxiliary battery (60) is greater than or equal to predicted driving power, the control unit (80) is configured to switch the power conversion unit (70) to the main power input so as to operate the UV driving unit (90).

2. The UV sterilization apparatus for the vehicle according to claim 1, further comprising an operation display unit (110) configured to display an operating state and result of the control unit (80).

3. The UV sterilization apparatus for the vehicle according to claim 1 or 2, wherein the UV driving unit (90) comprises a plurality of ports which allow driving power to be output differently according to the UV modules (100).

4. The UV sterilization apparatus for the vehicle according to any one of the claims 1 to 3, wherein the control unit (80) is configured to determine a case where an absence state of a passenger is maintained for more than a set time, as the operating condition based on any one or more of a starting state, a shifting state, a door opening/closing state, a door locking state, a seating state, an audio/video/navigation (AVN) state, an emergency light lighting state, a seat movement state, an fob signal state, and a rear seat detection state from the vehicle information.

5. The UV sterilization apparatus for the vehicle according to any one of the claims 1 to 4, wherein, when the control unit (80) determines the operating condition and then a charging state of the main battery is less than expected driving power, the control unit (80) is configured to switch the power conversion unit (70) to the auxiliary power so as to drive the UV driving unit (90).

6. The UV sterilization apparatus for the vehicle according to any one of the claims 1 to 5, wherein, when an abnormality occurs in the state of the main power input while the UV driving unit is operating, the control unit (80) is configured to switch the power conversion unit (70) to the auxiliary power so as to operate the UV driving unit (90).

7. The UV sterilization apparatus for the vehicle according to any one of the claims 1 to 6, wherein, when the control unit (80) determines the operating condition and then a charging state of the main battery is less than a set value, the control unit (80) is configured to stop an operation of the UV driving unit (90).

8. The UV sterilization apparatus for the vehicle according to any one of the claims 1 to 7, wherein, when a door lock is released while the UV driving unit (90) is operating, the control unit (80) is configured to stop the operation of the UV driving unit (90) and to temporarily stop counting of the sterilization driving time, and when the operating condition is satisfied again, the control unit (80) is configured to count the sterilization driving time so as to operate the UV driving unit (90) again.

9. The UV sterilization apparatus for the vehicle of claim 8, wherein, when the control unit (80) determines again whether the operating condition is satisfied and then the operating condition is not satisfied, the control unit (80) is configured to stop the operation of the UV driving unit (90) and to initialize counting.

10. The UV sterilization apparatus for the vehicle according to any one of the claims 1 to 9, wherein the sterilization driving time is set based on any one or more of a starting time, a door opening time, and temperature and humidity inside the vehicle.

## Patentansprüche

1. Ultraviolett- bzw. UV-Sterilisationsvorrichtung für ein Fahrzeug, umfassend:
eine Hauptleistung-Eingangseinheit (20), konfiguriert zum Empfangen von Hauptleistung von einer Hauptbatterie eines Fahrzeugs;
eine Hauptleistung-Detektionseinheit (10), konfiguriert zum Detektieren eines Status eines Hauptleistungseingangs von der Hauptleistung-Eingangseinheit (20);
eine Fahrzeuginformationen-Eingangseinheit (50), konfiguriert zum Empfangen von Fahrzeuginformationen zum Bestimmen eines Status des Fahrzeugs;
eine Vielzahl von UV-Modulen (100), dafür konfiguriert, an einer Vielzahl von Orten in dem Fahrzeug installiert zu werden, und konfiguriert zum Ausstrahlen von UV-Strahlen und Sterilisieren des Inneren des Fahrzeugs während einer Sterilisationsbetriebszeit;
eine UV-Antriebseinheit (90), konfiguriert zum Antreiben der Vielzahl von UV-Modulen (100); und
eine Steuereinheit (80), konfiguriert zum Empfangen eines Fahrzeuginformationeneingangs von der Fahrzeuginformationen-Eingangseinheit (50) und des Status des Hauptleistungseingangs und zum Bestimmen eines Betriebszustands der UV-Module (100),
**dadurch gekennzeichnet, dass** die UV-Sterilisationsvorrichtung ferner eine Zusatzleistung-Eingangseinheit (30) umfasst, konfiguriert zum Empfangen von Zusatzleistung von einer Zusatzbatterie (60), eine Zusatzleistung-Detektionseinheit (40), konfiguriert zum Detektieren eines Ladestatus der mit der Zusatzleistung-Eingangseinheit (30) verbundenen Zusatzbatterie (60), und eine Leistungsumwandlungseinheit (70), konfiguriert zum Umschalten zwischen dem Hauptleistungseingang und einem Zusatzleistungseingang von der Hauptleistung-Eingangseinheit (20) bzw. der Zusatzleistung-Eingangseinheit (30), wobei die Steuereinheit (80) ferner konfiguriert ist zum Empfangen des Ladestatus der Zusatzbatterie (60) und Steuern der Leistungsumwandlungseinheit (70), um die Vielzahl von UV-Modulen (100) unabhängig durch die UV-Antriebseinheit (90) während der Sterilisationsbetriebszeit anzutreiben, und
wenn der Status des Hauptleistungseingangs normal ist, nachdem die Steuereinheit (80) zum Bestimmen des Betriebszustands der UV-Module (100) konfiguriert ist, die Steuereinheit (80) ferner konfiguriert ist zum Bestimmen des Ladestatus der Zusatzbatterie (60), und wenn der Ladestatus der Zusatzbatterie (60) größer als eine oder gleich einer vorhergesagten Antriebsleistung ist, die Steuereinheit (80) konfiguriert ist zum Umschalten der Leistungsumwandlungseinheit (70) zu dem Hauptleistungseingang, um die UV-Antriebseinheit (90) zu betreiben.

2. UV-Sterilisationsvorrichtung für das Fahrzeug nach Anspruch 1, ferner umfassend eine Betriebsanzeigeeinheit (110), konfiguriert zum Anzeigen eines Betriebsstatus und Ergebnisses der Steuereinheit (80).

3. UV-Sterilisationsvorrichtung für das Fahrzeug nach Anspruch 1 oder 2, wobei die UV-Antriebseinheit (90) eine Vielzahl von Anschlüssen umfasst, die gestatten, eine Antriebsleistung verschieden gemäß den UV-Modulen (100) auszugeben.

4. UV-Sterilisationsvorrichtung für das Fahrzeug nach einem der Ansprüche 1 bis 3, wobei die Steuereinheit (80) konfiguriert ist zum Bestimmen eines Falls, in dem ein Abwesenheitsstatus eines Insassen länger als eine eingestellte Zeit beibehalten wird, als den Betriebszustand basierend auf einem oder mehreren eines Anlassstatus, eines Schaltstatus, eines Tür-Öffnen/Schließen-Status, eines Türverriegelungsstatus, eines Sitzplatzstatus, eines Audio/Video/Navigation- bzw. AVN-Status, eines Notlicht-Beleuchtungsstatus, eines Sitzbewegungsstatus, eines Fobsignalstatus und eines Rücksitz-Detektionsstatus aus den Fahrzeuginformationen.

5. UV-Sterilisationsvorrichtung für das Fahrzeug nach einem der Ansprüche 1 bis 4, wobei, wenn die Steuereinheit (80) den Betriebszustand bestimmt und dann ein Ladestatus der Hauptbatterie geringer als eine erwartete Antriebsleistung ist, die Steuereinheit (80) konfiguriert ist zum Umschalten der Leistungsumwandlungseinheit (70) zu der Zusatzleistung, um die UV-Antriebseinheit (90) zu betreiben.

6. UV-Sterilisationsvorrichtung für das Fahrzeug nach einem der Ansprüche 1 bis 5, wobei, wenn eine Anomalie in dem Status des Hauptleistungseingangs vorkommt, während die UV-Antriebseinheit (90) in Betrieb ist, die Steuereinheit (80) konfiguriert ist zum Umschalten der Leistungsumwandlungseinheit (70) zu der Zusatzleistung, um die UV-Antriebseinheit (90) zu betreiben.

7. UV-Sterilisationsvorrichtung für das Fahrzeug nach einem der Ansprüche 1 bis 6, wobei, wenn die Steuereinheit (80) den Betriebszustand bestimmt und dann ein Ladestatus der Hauptbatterie geringer als ein eingestellter Wert ist, die Steuereinheit (80) konfiguriert ist zum Stoppen eines Betriebs der UV-Antriebseinheit (90).

8. UV-Sterilisationsvorrichtung für das Fahrzeug nach einem der Ansprüche 1 bis 7, wobei, wenn eine Türverriegelung gelöst wird, während die UV-Antriebseinheit (90) in Betrieb ist, die Steuereinheit (80) konfiguriert ist zum Stoppen des Betriebs der UV-Antriebseinheit (90) und zum vorübergehenden Stoppen des Zählens der Sterilisationsbetriebszeit, und wenn der Betriebszustand wieder befriedigt wird, die Steuereinheit (80) konfiguriert ist zum Zählen der Sterilisationsbetriebszeit, um die UV-Antriebseinheit(90) wieder zu betreiben.

9. UV-Sterilisationsvorrichtung für das Fahrzeug nach Anspruch 8, wobei, wenn die Steuereinheit (80) wieder bestimmt, ob der Betriebszustand befriedigt wird und dann der Betriebszustand nicht befriedigt wird, die Steuereinheit (80) konfiguriert ist zum Stoppen des Betriebs der UV-Antriebseinheit (90) und zum Einleiten des Zählens.

10. UV-Sterilisationsvorrichtung für das Fahrzeug nach einem der Ansprüche 1 bis 9, wobei die Sterilisationsbetriebszeit basierend auf einem oder mehreren einer Anlasszeit, einer Türöffnungszeit und einer Temperatur und Luftfeuchtigkeit im Inneren des Fahrzeugs eingestellt wird.

## Revendications

1. Appareil de stérilisation aux ultraviolets, UV, pour un véhicule, comprenant :
une unité d'entrée de puissance principale (20) configurée pour recevoir une puissance principale d'une batterie principale d'un véhicule ;
une unité de détection de puissance principale (10) configurée pour détecter un état d'une entrée de puissance principale provenant de l'unité d'entrée de puissance principale (20) ;
une unité d'entrée d'informations de véhicule (50) configurée pour recevoir des informations de véhicule pour déterminer un état du véhicule ;
une pluralité de modules UV (100) configurés pour être installés à une pluralité d'emplacements dans le véhicule et configurés pour irradier des rayons UV et stériliser la partie intérieure du véhicule pendant un temps d'excitation pour stérilisation ;
une unité d'excitation UV (90) configurée pour exciter la pluralité de modules UV (100) ; et
une unité de commande (80) configurée pour recevoir une entrée d'informations de véhicule de l'unité d'entrée d'informations de véhicule (50) et l'état de l'entrée de puissance principale, et déterminer une condition de fonctionnement des modules UV (100),
**caractérisé en ce que** l'appareil de stérilisation aux UV comprend en outre une unité d'entrée de puissance auxiliaire (30) configurée pour recevoir une puissance auxiliaire d'une batterie auxiliaire (60), une unité de détection de puissance auxiliaire (40) configurée pour détecter un état de charge de la batterie auxiliaire (60) connectée à l'unité d'entrée de puissance auxiliaire (30), et une unité de conversion de puissance (70) configurée pour commuter entre l'entrée de puissance principale et une entrée de puissance auxiliaire provenant de l'unité d'entrée de puissance principale (20) et de l'unité d'entrée de puissance auxiliaire (30), respectivement, l'unité de commande (80) est en outre configurée pour recevoir l'état de charge de la batterie auxiliaire (60) et commander l'unité de conversion de puissance (70) de manière à exciter la pluralité de modules UV (100) indépendamment à travers l'unité d'excitation UV (90) pendant le temps d'excitation pour stérilisation, et
lorsque l'état de l'entrée de puissance principale est normal après que l'unité de commande (80) est configurée pour déterminer la condition de fonctionnement des modules UV (100), l'unité de commande (80) est en outre configurée pour déterminer l'état de charge de la batterie auxiliaire (60), et lorsque l'état de charge de la batterie auxiliaire (60) est supérieur ou égal à une puissance d'excitation prédite, l'unité de commande (80) est configurée pour commuter l'unité de conversion de puissance (70) sur l'entrée de puissance principale de manière à faire fonctionner l'unité d'excitation UV (90).

2. Appareil de stérilisation aux UV pour le véhicule selon la revendication 1, comprenant en outre une unité d'affichage de fonctionnement (110) configurée pour afficher un état de fonctionnement et un résultat de l'unité de commande (80).

3. Appareil de stérilisation aux UV pour le véhicule selon la revendication 1 ou 2, dans lequel l'unité d'excitation UV (90) comprend une pluralité de ports qui permettent à la puissance d'excitation d'être délivrée en sortie différemment selon les modules UV (100).

4. Appareil de stérilisation aux UV pour le véhicule selon l'une quelconque des revendications 1 à 3, dans lequel l'unité de commande (80) est configurée pour déterminer un cas où un état d'absence d'un passager est maintenu pendant plus d'un temps défini, comme la condition de fonctionnement sur la base d'un ou plusieurs parmi un état de démarrage, un état de changement de vitesse, un état d'ouverture/de fermeture de porte, un état de verrouillage de porte, un état d'assise, un état audio/vidéo/navigation (AVN), un état d'éclairage de lumière d'urgence, un état de mouvement de siège, un état de signal de télécommande et un état de détection de siège arrière à partir des informations de véhicule.

5. Appareil de stérilisation aux UV pour le véhicule selon l'une quelconque des revendications 1 à 4, dans lequel, lorsque l'unité de commande (80) détermine la condition de fonctionnement, puis un état de charge de la batterie principale est inférieur à la puissance d'excitation attendue, l'unité de commande (80) est configurée pour commuter l'unité de conversion de puissance (70) sur la puissance auxiliaire afin d'exciter l'unité d'excitation UV (90).

6. Appareil de stérilisation aux UV pour le véhicule selon l'une quelconque des revendications 1 à 5, dans lequel, lorsqu'une anomalie se produit dans l'état de l'entrée de puissance principale pendant que l'unité de d'excitation UV (90) fonctionne, l'unité de commande (80) est configurée pour commuter l'unité de conversion de puissance (70) sur la puissance auxiliaire afin de faire fonctionner l'unité d'excitation UV (90).

7. Appareil de stérilisation aux UV pour le véhicule selon l'une quelconque des revendications 1 à 6, dans lequel, lorsque l'unité de commande (80) détermine la condition de fonctionnement puis un état de charge de la batterie principale est inférieur à une valeur définie, l'unité de commande (80) est configurée pour arrêter le fonctionnement de l'unité d'excitation UV (90).

8. Appareil de stérilisation aux UV pour le véhicule selon l'une quelconque des revendications 1 à 7, dans lequel, lorsqu'un verrou de porte est libéré pendant que l'unité d'excitation UV (90) fonctionne, l'unité de commande (80) est configurée pour arrêter le fonctionnement de l'unité d'excitation UV (90) et pour arrêter temporairement le comptage du temps d'excitation pour stérilisation, et lorsque la condition de fonctionnement est à nouveau satisfaite, l'unité de commande (80) est configurée pour compter le temps d'excitation pour stérilisation de manière à faire fonctionner l'unité d'excitation UV (90) à nouveau.

9. Appareil de stérilisation aux UV pour le véhicule de la revendication 8, dans lequel, lorsque l'unité de commande (80) détermine à nouveau si la condition de fonctionnement est satisfaite, puis la condition de fonctionnement n'est pas satisfaite, l'unité de commande (80) est configurée pour arrêter le fonctionnement de l'unité d'excitation UV (90) et pour initialiser le comptage.

10. Appareil de stérilisation aux UV pour le véhicule selon l'une quelconque des revendications 1 à 9, dans lequel le temps d'excitation pour stérilisation est défini sur la base d'un ou plusieurs parmi un temps de démarrage, un temps d'ouverture de porte, et la température et l'humidité à l'intérieur du véhicule.
